# EUROPEAN PATENT SPECIFICATION

(11) **EP 0 859 796 B1**
(45) Date of publication and mention of the grant of the patent: **02.11.2000**
(21) Application number: 97937250.5
(22) Date of filing: 14.08.1997
(51) Int. Cl.: C08F 2/24, C11D 3/37, C09D 7/00

(54) **AQUEOUS COMPOSITIONS THICKENED WITH ACRYLATE-BASED POLYMERIC RHEOLOGY MODIFIERS**
WÄSSRIGE ZUSAMMENSETZUNGEN VERDICHT MIT POLYMEREN RHEOLOGIEMODIFIZIERERN AUF ACRYLATBASIS
COMPOSITIONS AQUEUSES EPAISSIES AVEC DES MODIFICATEURS RHEOLOGIQUES POLYMERIQUES A BASE D'ACRYLATE

(30) Priority: 16.08.1996 US 698690; 13.08.1997 US 910378
(43) Date of publication of application: 26.08.1998
(73) Proprietor: NATIONAL STARCH AND CHEMICAL INVESTMENT HOLDING CORPORATION, Wilmington, Delaware 19850 (US)
(72) Inventor: VERSTRAT, Daniel, W., Ooltewah, TN 37363 (US); BARRON, Milagros, C., Hixson, TN 37343 (US); WILKERSON, John, M., III, Hixson, TN 37343 (US)
(74) Representative: Hagemann, Heinrich, Dr.rer.nat., Dipl.-Chem.
(86) International application number: US9714317
(87) International publication number: WO9806757

(56) References cited:
- EP-A- 0 055 801
- EP-A- 0 671 157
- FR-A- 2 353 576
- US-A- 5 102 936
- US-A- 5 294 692

## Description

The present invention is related to acid- and/or surfactant-containing aqueous compositions having pH of less or equal to 11, more particularly from 0.4 to 10.5, which have been thickened with an acrylate-based polymeric rheology modifier.

Rheology modifiers are used generally to adjust or modify the rheological properties of aqueous compositions. Such properties include, without limitation, viscosity, flow rate, stability to viscosity change over time, and the ability to suspend particles in such aqueous compositions. The particular type of modifier used will depend on the particular aqueous composition to be modified and on the particular end-use of that modified aqueous composition. Examples of conventional rheology modifiers include thickeners such as cellulosic derivatives, polyvinyl alcohol, sodium polyacrylate, and other water-soluble macromolecules, and copolymeric emulsions in which monomers with acid groups have been introduced onto the main chain. Such thickeners are used widely in fiber treatment and adhesives.

It has been reported that when thickeners such as cellulosic derivatives and polyvinyl alcohol are mixed with aqueous emulsions, the thickened emulsion tends to exhibit poor stability to viscosity change over time. The cellulosics are said to result in a substantial decline in viscosity over time. It also has been reported that large quantities of polyvinyl alcohol are required in order to thicken aqueous emulsions. When such thickened aqueous emulsions are used in, for example, adhesives and coatings, the high levels of polyvinyl alcohol result in a loss of adhesive and/or cohesive properties as well as a loss in water resistance in the films formed therefrom

Another class of rheology modifiers known to thicken aqueous emulsions is one typically referred to as associative modifiers. Such associative modifiers are reported in U.S. Patent Nos. 4,743,698, 4,600,761, RE 33,156, 4,792,343, 4,384,096,3,657,175, 5,102,936 and 5,294,692. As noted, these thickeners become effective upon the addition of base, thereby raising the pH of the thickened composition to alkaline, but the thickeners are not designed to thicken aqueous compositions having a pH less than 7.

Other rheology modifiers which are "activated" by the addition of acid to aqueous compositions which contain the modifiers also have been reported. As reported, emulsions are prepared via free-radical emulsion polymerization utilizing colloidal stabilizers. The emulsions are mixed with the composition to be thickened and then acid is added to the mix, thereby lowering the pH of the system to 6.5 to 0.5 These thickeners are reported to be effective at thickening certain acidic aqueous compositions, but are not effective at thickening aqueous compositions having basic pH

It would be desirable to develop a rheology modifier which is stable to change in viscosity and phase separation over time, which does not detrimentally affect film properties such as adhesive/cohesive properties and water resistance, and which advantageously may be used to thicken both acidic and basic aqueous compositions.

The present invention relates to thickened aqueous compositions which have a pH of less than or equal to 11, preferably from 0.4 to 10.5, which compositions comprise a thickening amount of an acrylate-based polymeric rheology modifier in the form of a stable, aqueous emulsion; and an active ingredient selected from the group consisting of an acid and a surfactant, said active ingredient being present in amounts effective to provide to said thickened composition the performance characteristics required for the particular end-use thereof. The stable emulsion of the acrylate-based polymeric rheology modifier is prepared by single-stage emulsion polymerization of from 5 to 80 weight percent of an acrylate monomer (a) selected from the group consisting of a C₂-C₆ alkyl ester of acrylic acid and a C₁-C₆ alkyl ester of methacrylic acid, from 5 to 80 weight percent of a monomer (b) selected from the group consisting of a vinyl-substituted heterocyclic compound containing at least one of a nitrogen or sulfur atom, (meth)acrylamide, a mono- or di- (C₁-C₄)alkylamino (C₁-C₄)alkyl (meth)acrylate, a mono or di-(C₁-C₄)alkylamino (C₁-C₄)alkyl (meth)acrylamide, 0 to 2 weight percent of a cross-linking monomer (c); and 0 to 30 weight percent of an associative monomer (d), all percentages based on the total weight of monomer. The emulsion polymerization is conducted in the presence of water, a first surfactant in amounts effective to emulsify the polymer in the water, a free-radical initiator, and from 0.5 to 20 weight percent of an alcohol selected from the group consisting of a C₂-C₁₂ linear or branched monohydric alcohol and non-polymeric polyhydric alcohols, such as ethylene glycol, propylene glycol and glycerol, based on the total weight of the stable emulsion. The emulsion polymerization is conducted essentially in the absence of a polymeric colloidal stabilizer.

Exemplary thickened aqueous compositions of the present invention include, without limitation, cleansers such as toilet bowl cleaners, hard surface cleaners and liquid hand dishwashing detergents, drilling fluid additives, saturants for corrugated paper manufacture, adhesives, paints, inks, dyes and anti-stat coatings for paper. The emulsions are stable, meaning that no appreciable phase separation or change in viscosity is noted over time, for example one to five days at standard temperature and pressure, such that the emulsions may not be used to thicken aqueous compositions having pH of less than or equal to 11.

The acrylate monomers (a) are selected from the group consisting of esters prepared from acrylic acid and C₂-C₆ alcohols, such as ethyl or propyl alcohol, and esters prepared from methacrylic acid and C₁-C₆ alcohols. (Meth)acrylic acid is used herein to denote both acrylic acid and methacrylic acid. Preferred acrylate monomers comprise C₂-C₆ alkyl esters of acrylic acid. Even more preferred, the acrylate monomer is ethyl acrylate. From 5 to 80 weight percent of the acrylate monomer are used in preparing the composition of the present invention, based on total weight of monomer. Preferably from 15 to 70 weight percent of the acrylate monomer are used, based on total weight of monomer. More preferably, from 40 to 70 weight percent of the acrylate monomer are used.

Methyl acrylate should not be used in preparing the emulsions and is not included within the metes and bounds of this invention, as it has been found to result in emulsions which are unstable with respect to viscosity change over time. It was unexpected that polymers prepared in the absence of a polymeric colloidal stabilizer with ethyl acrylate provided stability to viscosity change over time when compared to polymers prepared in the absence of a polymeric colloidal stabilizer with methyl acrylate, as emulsions prepared with methyl acrylate were found to be unstable to viscosity change.

In addition to the acrylate ester (a), polymerized therewith is a monomer (b)selected from the group consisting of a vinyl-substituted heterocyclic compound containing at least one of a nitrogen or sulfur atom, (meth)acrylamide, a mono- or di- (C₁-C₄)alkylamino (C₁-C₄)alkyl (meth)acrylate, a mono or di-(C₁-C₄)alkylamino (C₁-C₄)alkyl (meth)acrylamide. Exemplary monomers include N,N-dimethylamino ethyl methacrylate, N,N-diethylamino ethyl acrylate, N,N-diethylamino ethyl methacrylate, N-t-butylamino ethyl acrylate, N-t-butylamino ethyl methacrylate, N,N-dimethylamino propyl acrylamide, N,N-dimethylamino propyl methacrylamide, N,N-diethylamino propyl acrylamide and N,N-diethylamino propyl methacrylamide. From 5 to 80 weight percent of the monomer are used in preparing the modifiers of the present invention, based on total weight of monomer. Preferably, from 10 to 70 weight percent of the monomer are used, based on total weight of monomer. More preferably, from 20 to 60 weight percent of the monomer are used.

In addition to the required monomers, monomers which provide cross-linking in the polymer may also be utilized in relatively low amounts, up to 3 weight percent, based on the total weight of monomer. When used, the cross-linking monomers preferably are used at levels of from 0.1 to 3 weight percent, based on total weight of monomer. Cross-linking monomers include multi-vinyl-substituted aromatic monomers, alicyclic monomers selected from the group consisting of cycloparrafins and cycloolefins, di-functional esters of phthalic acid, di-functional esters of methacrylic acid, multi-functional esters of acrylic acid, dienes, trienes, tetraenes, and N-methylene-Bis-acrylamide. Exemplary cross-linking monomers include divinylbenzene, trivinylbenzene, 1,2.4-tricinylcyclohexane, 1,5-hexadiene, 1,5,9-decatriene, 1,9-decadiene, and 1,5-heptadiene, di-allyl phthalate, ethylene glycol dimethacrylate, polyethylene glycol dimeth-acrylate, penta- and tetra-acrylates, and N-methylene-Bis-acrylamide The polyethylene glycol dimethacrylates are particularly preferred for thickening in acid aqueous compositions, as they tend to minimize turbidity.

In certain preferred embodiments, an associative monomer may be used to prepare the rheology modifiers, in amounts up to 30 weight percent, based on total weight of monomer. When used, the associative monomers preferably are used at levels ranging from 0.1 to 10 weight percent, based on total weight of monomer. Such monomers include those disclosed in U.S. patent Nos. 3,657,175, 4,384,096, 4,616,074, 4,743,698, 4,792,343, 5,011,978, 5,102,936, 5,294,692, and Re. 33,156, the contents of all which are hereby incorporated herein as if set forth in their entirety. Preferred associative monomers include the urethane reaction products of a monoethylenically unsaturated isocyanate and non-ionic surfactants comprising C₁-C₄ alkoxy-terminated, block copolymers of 1,2-butylene oxide and 1,2-ethylene oxide, as disclosed in U.S. Patent No. 5,294,692 (Barron et al.), an ethylenically unsaturated copolymerizable surfactant monomer obtained by condensing a nonionic surfactant with methylenesuccinic acid (also known as itaconic acid) as disclosed in U.S. 4,616,074 (Ruffner), a surfactant monomer selected from the urea reaction product of a monoethylenically unsaturated monoisocyanate with a nonionic surfactant having amine functionality as disclosed in U.S. 5,011,978 (Barron et al.), and a nonionic urethane monomer which is the urethane reaction product of a monohydric nonionic surfactant with a monoethylenically unsaturated monoisocyanate, preferably one lacking ester groups such as alpha, alpha-dimethyl-m-iso-propenyl benzyl isocyanate as disclosed in U.S. Re. 33,156 (Shay et al.). Particularly preferred are the ethylenically unsaturated copolymerizable surfactant monomers obtained by condensing a nonionic surfactant with methylenesuccinic acid. Methods for preparing such monomers are disclosed in detail in the various patents incorporated herein above.

The rheology modifier is prepared first by forming an emulsion utilizing single-stage emulsion polymerization techniques. Monomer, water, free-radical initiator, a first surfactant in amounts effective to disperse the polymer in the water upon polymerization of the monomers, and from 0.5 to 20 weight percent of an alcohol selected from the group consisting of a C₂-C₁₂ linear or branched monohydric alcohol and a non-polymeric polyhydric alcohol, such as ethylene glycol, propylene glycol and glycerol, based on total weight of the emulsion, are combined in a polymerization reactor and maintained at a desired temperature and for a period of time which are effective to polymerize the monomers, thereby forming a polymeric emulsion comprising the copolymer of monomers (a) and (b), water, surfactant and alcohol.

The contents of the polymerization vessel preferably are maintained at a temperature and for a period of time effective to cause polymerization of the monomers. Preferably the polymerization reaction is initiated at about 30 degrees Centrigrade, with the contents of the polymerization vessel attaining a temperature of about 60 degrees Centigrade. The reaction time will be from 1 to 6 hours. One skilled in the art of emulsion polymerization will be able to ascertain readily exactly what conditions of temperature and time are required, as both are well within the knowledge of one skilled in the art.

Preferably, from 1 to 10 weight percent of the alcohol are used and, more preferably, from 1 to 5 weight percent of the alcohol are used, based on the total weight of the emulsion. If no alcohol, or an insufficient amounts of the alcohol, is used in preparing the emulsion, the resultant emulsion will not be stable to change in viscosity over time. It is desirable to minimize the level of alcohol used. The maximum amount of alcohol used may be limited practically by factors such as cost, flammability and volatile organic compound environmental concerns. Other than those factors, amounts of alcohol in excess of 20 weight percent conceivably may used.

It is essential that polymeric colloidal stabilizers such as polyvinyl alcohol not be used during preparation of the emulsion via emulsion polymerization in any amount which materially alters the properties of the emulsion, particularly the emulsion stability. Preferably, no polymeric colloidal stabilizer is used during emulsion preparation. It was discovered surprisingly that use of such polymeric colloidal stabilizers results in emulsions which are not stable to changes in viscosity or phase separation over time. Accordingly, the emulsions and rheology modifiers comprising the emulsions essentially are free of polymeric colloidal stabilizers.

Thickened aqueous compositions according to the present invention, in addition to containing a thickening amount of the acrylate-based, stable aqueous emulsion, that is, an amount effective to thicken the aqueous composition compared to a comparable composition which does not contain the acrylate-based emulsion, will also comprise a minimum effective amount of an active ingredient selected from the group consisting of an acid and a second surfactant. Preferably, the thickened composition will comprise enough of the emulsion such that the thickened composition comprises greater than 0.5 dry weight percent of the emulsion polymer on a dry weight basis, based on total weight of the thickened aqueous composition, and more preferably from 0.5 to 20 dry weight percent of the polymer, based on total weight of the thickened composition. Most preferably, the thickened composition will comprise from 1 to 10 dry weight percent of the emulsion polymer, based on total weight of the thickened composition.

By minimum effective amount, it is meant that the active ingredient will be present in the thickened aqueous composition in a minimum amount effective to provide the thickened aqueous composition with the particular performance characteristics required for the particular end-use of the thickened composition. For example, where the thickened composition is a cleanser, the composition must provide cleaning performance, as described below. In some cases, the surfactant may be used to enhance the thickening efficiency of the acrylate-based emulsion, for instance in an ink paste base or in an acid-containing cleanser. Accordingly, the thickened composition may comprise from 0.1 to 95 active weight percent of surfactant and/or from 0.1 to 50 active weight percent acid, based on total weight of the thickened composition, with the balance being polymer and water, as well as possibly other optional conventional ingredients utilized in thickened compositions described herein.

The actual active ingredient and the actual minimum effective amount will be determined by the actual product/application in which the thickened composition is to be used. For example, where the end-use is a cleaning composition such as a toilet bowl cleaner, a hard surface cleaner or a liquid hand dishwashing detergent, the active ingredient is selected from the group consisting of an acid and a second surfactant, present at a minimum amount effective to achieve minimum cleansing performance. By minimum cleansing performance, it is meant that the active ingredient is present in minimum amounts effective to clean or remove deposits from the surface of substrates to which the thickened aqueous compositions have been applied. For example, where the composition is applied to toilet bowls, an acid will be present in minimum amounts effective to remove salts and stains caused by continuous and/or repeated exposure to water, for example iron salts such as rust and the like. In this case, the thickened composition may comprise from 0.1 to 50 active weight percent of the acid, more preferably from 2 to 50 active weight percent of acid, based on total weight of the thickened composition

Where the compositions are applied in the form of a liquid hand dishwashing detergent to, for instance, dishes and plates, a second surfactant will be present in minimum amounts effective to remove deposits such as oils and fatty substances emanating from food products, dried food products themselves, dirt, and so forth. Preferably, such cleaning composition will comprise from 2 to 95 active weight percent of the second surfactant, more preferably from 5 to 95 active weight percent of the second surfactant, based on total weight of the thickened aqueous composition.

Exemplary acids used in compositions of the present invention include, without limitation, citric, sulfuric, hydrochloric, phosphoric, acetic, hydroxyacetic, and sulfamic acids. Synergistic thickening of compositions comprising an amount of an acid required for a desired performance have been surprisingly noted where a second surfactant selected from the group consisting of a nonionic and anionic surfactant is added to the composition. In those cases, the addition of the second surfactant has been found to improve thickening efficiency by as much as eighty fold, depending on the amount of surfactant added to the aqueous composition which comprises an acid. In these cases, the second surfactant need not be present at a level greater than that necessary to impart synergistic thickenening of the composition, as the acid performs the primary application function and the second surfactant serves to improve the thickening efficiency of the acrylate-based emulsion.

Thickened aqueous compositions according to the invention also include those compositions which comprise a second surfactant in minimum cleansing amounts. Such thickened compositions include, for example, liquid hand dishwashing detergents. Exemplary second surfactants which may be used in the thickened aqueous compositions include, without limitation, sodium lauryl sulfate, (di)alkylsulfosuccinates, alkyl sulfonates, alkyl phosphates, alkyl ethoxylates, and alkylaryl ethoxylates It should be noted that the second surfactant is in addition to the first surfactant, which will be present in the aqueous composition due to its presence in the acrylate-based emulsion. Accordingly, the second surfactant may be formulated or blended into the aqueous composition after the emulsion has been prepared, and should not be confused with the first surfactant. While the second surfactant may be the same as the first surfactant, it is preferred that the second surfactant be selected from the group consisting of anionic and nonionic surfactants, and thus may be different than the first surfactant used to prepare the emulsion

In addition to the above essential elements of the invention, the thickened aqueous compositions may further comprise conventional ingredients known to be used therein

The following examples are set forth merely to exemplify the invention and are not intended to limit the metes and bounds of the invention, which is set forth by the claims appended hereto.

### Example 1: Acid Thickening

Two emulsions were prepared according to procedures set forth herein above and designated Emulsions 1A and 1B respectively Each emulsion was prepared at twenty (20) weight percent polymer solids, based on total weight of the emulsion, utilizing acrylate-ester and alkylamino (meth)acrylate monomers. Each emulsion was incorporated into an acid-containing solution at a level such that the polymer solids, based on total weight of the thickened composition, are as set forth in Table 1.

**TABLE 1**

| **Emulsion sample** ^{**[2]**} | | **Brookfield Viscosity** ^{**[1]**} **(cPs)** | | | |
|---|---|---|---|---|---|
| | **pH** | **initial** | **1 day** | **5 day** | **35 day** |
| **Emulsion 1A (20% polymer solids)** | 8.2 | 50 | 60 | 66 | 92 |
| 3% 1A in 5% citric acid | 2.2 | 3200 | 6000 | 6000 | 5300 |
| 3% 1A in 5% hydroxyacetic / 5% sulfamic acid blend | 2.2 | 2200 | 4500 | 4000 | 4400 |
| 4% 1A in 9% hydrochloric acid | 0.4 | 740 | 2060 | 1900 | ------- |
| **Emulsion 1B (20% polymer solids)** | 8.2 | 108 | 280 | 310 | 1550 |
| 3% 1 B in 5% citric acid | 2.2 | 6000 | 8000 | 8700 | 8550 |
| 3% 1B in 5% hydroxyacetic / 5% sulfamic acid blend | 2.3 | 4150 | 7000 | 6800 | 6800 |

| | | | | | |
|---|---|---|---|---|---|
| [1] Brookfield viscometer model RVDVII+ ; All measurements obtained with spindle #2 @ 20 rpm, 21°C. | | | | | |
| [2] All values are listed in dry weight % on total composition weight; e.g. 3 % 1A polymer solids and 5 % citric acid solids, based on total weight of thickened composition. | | | | | |

### Example 2: Surfactant Thickening

Stable emulsion 1A was added at the noted polymer solids to a blend of anionic and nonionic surfactants (2(a)) typical of commercially available liquid hand dishwashing detergents. The pH and Brookfield viscosity of the surfactant blend were recorded both prior to and after addition of the stable emulsion. The surfactant composition pH was then adjusted with addition of 50% sodium hydroxide solution to a pH 10 and the resulting Brookfield viscosity was recorded Again, the stable emulsion product unexpectedly provided a significant increase in the viscosity of the surfactant blend at pH 8.4 as well as pH 10.

Additional samples of anionic and non-ionic surfactants were selected for investigation of the ability of the stable emulsion thickener representative of this invention to thicken aqueous compositions comprising a second surfactant as the active ingredient. It is significant to note that the compositions of this example contain no acidic components, and no acid substances are added as neutralizing agents, compatibilizers, stablizers, or the like. The data contained in TABLE 2 includes as-is viscosity and pH measurements for an anionic surfactant (sodium lauryl sulfate, 2(d)) and a non-ionic surfactant (nonylphenol 4 mole ethoxylate (2(f)). The table also provides the resulting viscosity and pH of the referenced surfactant samples after addition of a noted level of the stable emulsion thickener 1A of this invention, demonstrating the ability of the stable emulsion thickener to provide an unexpected and substantial increase in the surfactant viscosity at an alkaline pH.

**TABLE 2**

| Sample # | Description | pH | Brookfield Viscosity ^{[2]} (cPs) |
|---|---|---|---|
| 2(a) | Commercial Anionic / Nonionic surfactant blend | 8.2 | 540 |
| 2(b) | Sample 2(a) with 1.2 % Emulsion 1A added ^{[1]} | 8.4 | 1270 |
| 2(c) | Sample 2(b) with added sodium hydroxide | 10.0 | 930 |
| 2(d) | Sodium lauryl sulfate (30 % active in water) | 8.6 | 94 |
| 2(e) | Sample 2(d) with 1.25 % 1A emulsion added ^{[1]} | 9.3 | 738 |
| 2(f) | Nonylphenol ethoxylate | 9.3 | 386 |
| 2(g) | Sample 2(f) with 2.2 % 1A emulsion added ^{[1]} | 8.1 | 1060 |

| | | | |
|---|---|---|---|
| [1] dry polymer weight % on total composition weight, | | | |
| [2] spindle #2 @ 20 rpm, 21°C. | | | |

### Example 3:Thickening Surfactant Containing Acidic Systems

Evaluation of the interaction of the stable emulsion product in an acidic system containing surfactant provided the data in TABLE 3, below. A solution containing 5 active weight % of citric acid was prepared. Addition of the stable emulsion product at a level of 2 % dry polymer on total solution weight yielded a significant increase in the citric acid solution Brookfield viscosity. To the thickened acid solution was added, in 2 gram increments, an ethoxylated alkylphenol surfactant (HLB 8.8). Viscosity response to the incremental addition of the nonionic surfactant was recorded and demonstrates the ability of the stable emulsion product to thicken systems containing both acid and surfactant. Increasing the HLB of the added surfactant over the surfactant referenced in TABLE 3 is expected to provide an initial increase in the system viscosity that will more rapidly decrease with continued addition of the surfactant versus the referenced TABLE 3 viscosity data.

**TABLE 3**

| Sample # | Sample Description | Brookfield Viscosity (cPs) ^{[1]} |
|---|---|---|
| 3(a) | 5 % Citric acid^{[2]} solution | <10 |
| 3(b) | 3(a) + 2 %1A emulsion^{[2]} | 216 |
| 3(c) | 3(b) + 2 grams nonionic surfactant | 4460 |
| 3(d) | 3(b) + 4 grams nonionic surfactant | 6200 |
| 3(e) | 3(b) + 6 grams nonionic surfactant | 8000 |
| 3(f) | 3(b) + 8 grams nonionic surfactant | 11200 |
| 3(g) | 3(b) + 10 grams nonionic surfactant | 16000 |
| 3(h) | 3(b) + 12 grams nonionic surfactant | 16000 |
| 3(i) | 3(b) + 14 grams nonionic surfactant | 9500 |
| 3(j) | 3(b) + 16 grams nonionic surfactant | 7200 |
| 3(k) | 3(b) + 18 grams nonionic surfactant | 6110 |
| 3(l) | 3(b) + 20 grams nonionic surfactant | 5000 |

| | | |
|---|---|---|
| [1] Brookfield Viscometer model RVDVII+; All measurements obtained with spindle #2 @ 20 rpm, 21°C. | | |
| [2] Solids weight percent based on total weight of solution. | | |

## Claims

1. An aqueous composition having a pH of less than or equal to 11, comprising:
a thickening amount of a stable emulsion of a polymer, said emulsion prepared by single-stage emulsion polymerization of monomers selected from the group consisting of
from 5 to 80 weight percent of an acrylate monomer (a) selected from the group consisting of a C₂-C₆ alkyl ester of acrylic acid and a C₁-C₆ alkyl ester of methacrylic acid,
from 5 to 80 weight percent of a monomer (b) selected from the group consisting of a vinyl-substituted heterocyclic compound containing at least one of a nitrogen or sulfur atom, (meth)acrylamide, a mono- or di- (C₁-C₄)alkylamino (C₁-C₄)alkyl (meth)acrylate, a mono or di-(C₁-C₄)alkylamino (C₁-C₄)alkyl (meth)acrylamide,
0 to 2 weight percent of a cross-linking monomer (c), and
0 to 30 weight percent of an associative monomer (d),
all percentages based on the total weight of monomer,
wherein said emulsion polymerization is conducted in the presence of water, a first surfactant in amounts effective to emulsify the polymer in the water, a free-radical initiator and from 0.5 to 20 weight percent of an alcohol selected from the group consisting of a C₂-C₁₂ linear or branched monohydric alcohol and a non-polymeric polyhydric alcohol, based on the total weight of said emulsion, wherein said single-stage emulsion polymerization is conducted essentially in the absence of a polymeric colloidal stabilizer; and
a minimum effective amount of an active ingredient selected from the group consisting of an acid and a second surfactant

2. The composition of Claim 1 wherein said emulsion comprises from 15 to 40 weight percent of said polymer, based on the total weight of said emulsion.

3. The composition of Claim 1 wherein said emulsion comprises from 0.1 to 5 weight percent of said first surfactant, based on the total weight of monomer, said first surfactant selected from the group consisting of anionic, cationic, nonionic, amphoteric and zwitterionic surfactants.

4. The composition of Claim 1 wherein said cross-linking monomer is selected from the group consisting of multi-vinyl-substituted aromatic monomers, alicyclic monomers selected from the group consisting of cycloparrafins and cycloolefins, di-functional esters of phthalic acid, di-functional esters of methacrylic acid, multi-functional esters of acrylic acid, dienes, trienes, tetraenes, and N-methylene-Bis-acrylamide.

5. The composition of Claim 4 wherein said emulsion comprises from 0.1 to 1 weight percent of said cross-linking monomer, based on the total weight of monomer.

6. The composition of Claim 1 wherein said associative monomer is selected from the group consisting of urethane reaction products of a monoethylenically unsaturated isocyanate and non-ionic surfactants comprising C₁-C₄ alkoxy-terminated, block copolymers of 1,2-butylene oxide and 1,2-ethylene oxide, an ethylenically unsaturated copolymerizable surfactant monomer obtained by condensing a nonionic surfactant with methylenesuccinic, a surfactant monomer selected from the urea reaction product of a monoethylenically unsaturated monoisocyanate with a nonionic surfactant having amine functionality), and a nonionic urethane monomer which is the urethane reaction product of a monohydric nonionic surfactant with a monoethylenically unsaturated isocyanate.

7. The composition of Claim 6 wherein said emulsion comprises from 0.1 to 10 weight percent of said associative monomer, based on total weight of monomer.

8. The composition of Claim 1 wherein the monomer (b) is selected from the group consisting of N,N-dimethylamino ethyl methacrylate, N,N-diethylamino ethyl acrylate, N,N-diethylamino ethyl methacrylate, N-t-butylamino ethyl acrylate, N-t-butylamino ethyl methacrylate, N,N-dimethylamino propyl acrylamide, N.N-dimethylamino propyl methacrylamide, N,N- diethylamino propyl acrylamide and N.N-diethylamino propyl methacrylamide.

9. The composition of Claim 1 wherein said second surfactant is selected from the group consisting of anionic and nonionic surfactants.

10. The composition of Claim 9 wherein the second surfactant is present in a minimum cleansing amount, up to 95 active weight percent, based on the total weight of said composition.

11. The composition of Claim 10 comprising from 2 to 95 active weight percent of said second surfactant, based on total weight of the thickened composition.

12. The composition of Claim 1 comprising from 0.1 to 95 active weight percent of said second surfactant, based on total weight of the thickened composition.

13. The composition of Claim 1 wherein the acid is present in a minimum cleansing amount, up to 50 active weight percent, based on the total weight of said composition.

14. The composition of Claim 13 comprising from 2 to 50 active weight percent of said acid, based on the total weight of said composition.

15. The composition of Claim 13 comprising said second surfactant in amounts effective to improve the thickening efficiency of said emulsion.

16. The composition of Claim 1 comprising from 0.1 to 50 active weight percent of said acid, based on the total weight of said composition.

17. The composition of Claim 1 comprising from 0.5 to 20 dry weight percent of said polymer, based on total weight of said composition.

18. The composition of Claim 1 wherein said acid is selected from the group consisting of citric, sulfuric, hydrochloric, phosphoric, acetic, hydroxyacetic and sulfamic acids.

19. The composition of Claim 1 wherein said second surfactant is selected from the group consisting of sodium lauryl sulfate, (di)alkylsulfosuccinates, alkyl sulfonates, alkyl phosphates, alkyl ethoxylates and alkylaryl ethoxylates.

20. A composition for use as a rheology modifier in aqueous systems having pH of less than or to 11, said composition comprising:
a stable emulsion of a polymer, said emulsion prepared by single-stage emulsion polymerization of monomers selected from the group consisting of
from 5 to 80 weight percent of an acrylate monomer (a) selected from the group consisting of a C₂-C₆ alkyl esters of acrylic acid and a C₁-C₆ alkyl ester of methacrylic acid,
from 5 to 80 weight percent of a monomer (b) selected from the group consisting of a vinyl-substituted heterocyclic compound containing at least one of a nitrogen or sulfur atom, (meth)acrylamide, a mono- or di- (C₁-C₄)alkylamino (C₁-C₄)alkyl (meth)acrylate, a mono or di-(C₁-C₄)alkylamino (C₁-C₄)alkyl (meth)acrylamide,
0 to 2 weight percent of a cross-linking monomer (c); and
0 to 30 weight percent of an associative monomer (d),
all percentages based on the total weight of monomer,
wherein said emulsion polymerization is conducted in the presence of water, a surfactant in amounts effective to emulsify the polymer in the water, a free-radical initiator, and from 0.5 to 20 weight percent of an alcohol selected from the group consisting of a C₂-C₁₂ linear or branched monohydric alcohol and a non-polymeric polyhydric alcohol, based on the total weight of said emulsion, wherein said emulsion polymerization is conducted essentially in the absence of a polymeric colloidal stabilizer.

21. The composition of Claim 20 wherein said emulsion comprises from 15 to 40 weight percent of said polymer, based on the total weight of said emulsion.

22. The composition of Claim 20 wherein said emulsion comprises from 0.1 to 5 weight percent of said surfactant, based on the total weight of monomer, said surfactant selected from the group consisting of anionic, cationic, nonionic, amphoteric and zwitterionic surfactants.

23. The composition of Claim 20 wherein said cross-linking monomer is selected from the group consisting of multi-vinyl-substituted aromatic monomers, alicyclic monomers selected from the group consisting of cycloparrafins and cycloolefins, di-functional esters of phthalic acid, di-functional esters of methacrylic acid, multi-functional esters of acrylic acid, dienes, trienes, tetraenes, and N-methylene-Bis-acrylamide.

24. The composition of Claim 23 wherein said emulsion comprises from 0.1 to 1 weight percent of said cross-linking monomer, based on the total weight of monomer.

25. The composition of Claim 20 wherein said associative monomer is selected from the group consisting of urethane reaction products of a monoethylenically unsaturated isocyanate and non-ionic surfactants comprising C₁-C₄ alkoxy-terminated, block copolymers of 1,2-butylene oxide and 1,2-ethylene oxide, an ethylenically unsaturated copolymerizable surfactant monomer obtained by condensing a nonionic surfactant with methylenesuccinic, a surfactant monomer selected from the urea reaction product of a monoethylenically unsaturated monoisocyanate with a nonionic surfactant having amine functionality), and a nonionic urethane monomer which is the urethane reaction product of a monohydric nonionic surfactant with a monoethylenically unsaturated monoisocyanate.

26. The composition of Claim 20 wherein said emulsion comprises from 0.1 to 10 weight percent of said associative monomer, based on total weight of monomer.

27. The composition of Claim 20 wherein the monomer (b) is selected from the group consisting of N,N-dimethylamino ethyl methacrylate, N,N-diethylamino ethyl acrylate, N,N-diethylamino ethyl methacrylate, N-t-butylamino ethyl acrylate, N-t-butylamino ethyl methacrylate, N,N-dimethylamino propyl acrylamide, N.N-dimethylamino propyl methacrylamide, N,N- diethylamino propyl acrylamide and N.N-diethylamino propyl methacrylamide.

28. The composition of Claim 20 consisting essentially of said stable emulsion

## Patentansprüche

1. Wäßrige Zusammensetzung mit einem pH-Wert von 11 oder weniger, umfassend:
eine verdickende Menge einer stabilen Emulsion eines Polymers, wobei die Emulsion durch einstufige Emulsionspolymerisation von Monomeren, ausgewählt aus der Gruppe, bestehend aus
5 bis 80 Gew.-% eines Acrylatmonomers (a), ausgewählt aus der aus einem C₂-C₆-Alkylester einer Acrylsäure und einem C₁-C₆-Alkylester einer Methacrylsäure bestehenden Gruppe,
5 bis 80 Gew.-% eines Monomers (b), ausgewählt aus der Gruppe, bestehend aus einer Vinyl-substituierten heterocyclischen Verbindung, enthaltend mindestens ein Stickstoff- oder Schwefelatom, (Meth)acrylamid, einem Mono- oder Di(C₁-C₄)alkylamino(C₁-C₄)alkyl(meth)acrylat, einem Mono- oder Di(C₁-C₄)alkylamino(C₁-C₄)alkyl(meth)acrylamid,
0 bis 2 Gew.-% eines vernetzenden Monomers (c),
und
0 bis 30 Gew.-% eines assoziativen Monomers (d), hergestellt wird,
wobei sämtliche Prozente auf das Gesamtgewicht an Monomer bezogen sind,
worin die Emulsionspolymerisation in Gegenwart von Wasser, einem ersten oberflächenaktiven Mittel in einer wirksamen Menge, um das Polymer in Wasser zu emulgieren, einem radikalischen Initiator und 0,5 bis 20 Gew.-% eines Alkohols, ausgewählt aus der aus einem linearen oder verzweigten einwertigen C₂-C₁₂-Alkohol und einem nicht-polymeren mehrwertigen Alkohol bestehenden Gruppe, bezogen auf das Gesamtgewicht der Emulsion, durchgeführt wird, worin die einstufige Emulsionspolymerisation im wesentlichen ohne einen polymeren kolloidalen Stabilisator und
einer minimal wirksamen Menge eines aktiven Bestandteils, ausgewählt aus der aus einer Säure und einem zweiten oberflächen Mittel bestehenden Gruppe, durchgeführt wird.

2. Zusammensetzung nach Anspruch 1, worin die Emulsion 15 bis 40 Gew.-% des Polymers, bezogen auf das Gesamtgewicht der Emulsion, umfasst.

3. Zusammensetzung nach Anspruch 1, worin die Emulsion 0,1 bis 5 Gew.-% des ersten oberflächenaktiven Mittels, bezogen auf das Gesamtgewicht an Monomer, umfasst, wobei das erste oberflächenaktive Mittel ausgewählt ist aus der aus anionischen, kationischen, nichtionischen, amphoteren und zwitterionischen oberflächenaktiven Mitteln bestehenden Gruppe.

4. Zusammensetzung nach Anspruch 1, worin das vernetzende Monomer ausgewählt ist aus der aus mehrfach Vinyl-substituierten aromatischen Monomeren, alicyclischen Monomeren, ausgewählt aus der aus Cycloparaffinen und Cycloolefinen, difunktionellen Estern von Phthalsäure, difunktionellen Estern von Methacrylsäure, polyfunktionellen Estern von Acrylsäure, Dienen, Trienen, Tetraenen und N-Methylen-bis-acrylamid bestehenden Gruppe.

5. Zusammensetzung nach Anspruch 4, worin die Emulsion 0,1 bis 1 Gew.-% des vernetzenden Monomers, bezogen auf das Gesamtgewicht an Monomer, umfasst.

6. Zusammensetzung nach Anspruch 1, worin das assoziative Monomer ausgewählt ist aus der Gruppe, bestehend aus Urethan-Umsetzungsprodukten eines monoethylenisch ungesättigten Isocyanats und nichtionischen oberflächenaktiven Mitteln, umfassend C₁-C₄-Alkoxy-terminierten, Block-Copolymeren von 1,2-Butylenoxid und 1,2-Ethylenoxid, einem ethylenisch ungesättigten copolymerisierbaren oberflächenaktiven Monomer, erhalten durch Kondensieren eines nichtionischen oberflächenaktiven Mittels mit Methylensuccinsäure, einem oberflächenaktiven Monomer, ausgewählt aus dem Harnstoff-Umsetzungsprodukt eines monoethylenisch ungesättigten Monoisocyanats mit einen nichtionischen oberflächenaktiven Mittel mit einer Amin-Funktionalität und ein nichtionisches Urethanmonomer, welches das Urethan-Umsetzungsprodukt eines einwertigen nichtionischen oberflächenaktiven Mittels mit einem monoethylenisch ungesättigten Isocyanat darstellt.

7. Zusammensetzung nach Anspruch 6, worin die Emulsion 0,1 bis 10 Gew.-% des assoziativen Monomers, bezogen auf das Gesamtgewicht an Monomer, umfasst.

8. Zusammensetzung nach Anspruch 1, worin das Monomer (b) ausgewählt ist aus der aus N,N-Dimethylaminoethylmethacrylat, N,N-Diethylaminoethylacrylat, N,N-Diethylaminoethylmethacrylat, N-t-Butylaminoethylacrylat, N-t-Butylaminoethylmethacrylat, N,N-Dimethylaminopropylacrylamid, N,N-Dimethylaminopropylmethacrylamid, N,N-Diethylaminopropylacrylamid und N,N-Diethylaminopropylmethacrylamid bestehenden Gruppe.

9. Zusammensetzung nach Anspruch 1, worin das zweite oberflächenaktive Mittel ausgewählt ist aus der aus anionischen und nichtionischen oberflächenaktiven Mitteln bestehenden Gruppe.

10. Zusammensetzung nach Anspruch 9, worin das zweite oberflächenaktive Mittel in einer minimalen Reinigungsmenge bis zu 95 wirksame Gew.-%, bezogen auf das Gesamtgewicht der Zusammensetzung, vorliegt.

11. Zusammensetzung nach Anspruch 10, umfassend 2 bis 95 wirksame Gew.-% des zweiten oberflächenaktiven Mittels, bezogen auf das Gesamtgewicht der eingedickten Zusammensetzung.

12. Zusammensetzung nach Anspruch 1, umfassend 0,1 bis 95 wirksame Gew.-% des zweiten oberflächenaktiven Mittels, bezogen auf das Gesamtgewicht der eingedickten Zusammensetzung.

13. Zusammensetzung nach Anspruch 1, worin die Säure in einer minimalen Reinigungsmenge bis zu 50 wirksamen Gew.-%, bezogen auf das Gesamtgewicht der Zusammensetzung, vorliegt.

14. Zusammensetzung nach Anspruch 13, umfassend 2 bis 50 wirksame Gew.-% der Säure, bezogen auf das Gesamtgewicht der Zusammensetzung.

15. Zusammensetzung nach Anspruch 13, umfassend das zweite oberflächenaktive Mittel in einer wirksamen Menge, um die Verdickungseffizienz der Emulsion zu verbessern.

16. Zusammensetzung nach Anspruch 1, umfassend 0,1 bis 50 wirksame Gew.-% der Säure, bezogen auf das Gesamtgewicht der Zusammensetzung.

17. Zusammensetzung nach Anspruch 1, umfassend 0,5 bis 20 Gew.-% trockenes Polymer, bezogen auf das Gesamtgewicht der Zusammensetzung.

18. Zusammensetzung nach Anspruch 1, worin die Säure ausgewählt ist aus der aus Zitronensäure, Schwefelsäure, Salzsäure, Phosphorsäure, Essigsäure, Hydroxyessigsäure und Sulfaminsäure gebildeten Gruppe.

19. Zusammensetzung nach Anspruch 1, worin das zweite oberflächenaktive Mittel ausgewählt ist aus der aus Natriumlaurylsulfat, (Di)alkylsulfosuccinaten, Alkylsulfonaten, Alkylphosphaten, Alkylethoxylaten und Alkylarylethoxylaten bestehenden Gruppe.

20. Zusammensetzung zur Verwendung als RheologieModifizierer in wäßrigen Systemen mit einem pH-Wert von 11 oder kleiner, wobei die Zusammensetzung umfasst:
eine stabile Emulsion eines Polymers, wobei die Emulsion durch einstufige Emulsionspolymerisation von Monomeren, ausgewählt aus der Gruppe, bestehend aus
5 bis 80 Gew.-% eines Acrylatmonomers (a), ausgewählt aus der aus einem C₂-C₆-Alkylester einer Acrylsäure und einem C₁-C₆-Alkylester einer Methacrylsäure bestehenden Gruppe,
5 bis 80 Gew.-% eines Monomers (b), ausgewählt aus der Gruppe, bestehend aus einer Vinyl-substituierten heterocyclischen Verbindung, enthaltend mindestens ein Stickstoff- oder Schwefelatom, (Meth)acrylamid, einem Mono- oder Di(C₁-C₄)alkylamino(C₁-C₄)alkyl(meth)acrylat, einem Mono- oder Di(C₁-C₄)alkylamino(C₁-C₄)alkyl(meth)acrylamid,
0 bis 2 Gew.-% eines vernetzenden Monomers (c),
und
0 bis 30 Gew.-% eines assoziativen Monomers (d), hergestellt wird,
wobei sämtliche Prozente auf des Gesamtgewicht an Monomer bezogen sind,
worin die Emulsionspolymerisation in Gegenwart von Wasser, einem oberflächenaktiven Mittel in einer wirksamen Menge, um das Polymer in Wasser zu emulgieren, einem radikalischen Initiator und 0,5 bis 20 Gew.-% eines Alkohols, ausgewählt aus der aus einem linearen oder verzweigten einwertigen C₂-C₁₂-Alkohol und einem nicht-polymeren mehrwertigen Alkohol bestehenden Gruppe, bezogen auf das Gesamtgewicht der Emulsion, durchgeführt wird, worin die Emulsionspolymerisation im wesentlichen ohne einen polymeren kolloidalen Stabilisator durchgeführt wird.

21. Zusammensetzung nach Anspruch 20, worin die Emulsion 15 bis 40 Gew.-% des Polymers, bezogen auf das Gesamtgewicht der Emulsion, umfasst.

22. Zusammensetzung nach Anspruch 20, worin die Emulsion 0,1 bis 5 Gew.-% des ersten oberflächenaktiven Mittels, bezogen auf das Gesamtgewicht an Monomer, umfasst, wobei das oberflächenaktive Mittel ausgewählt ist aus der aus anionischen, kationischen, nichtionischen, amphoteren und zwitterionischen oberflächenaktiven Mitteln bestehenden Gruppe.

23. Zusammensetzung nach Anspruch 20, worin das vernetzende Monomer ausgewählt ist aus der Gruppe, bestehend aus mehrfach Vinyl-substituierten aromatischen Monomeren und alicyclischen Monomeren, ausgewählt aus der aus Cycloparaffinen und Cycloolefinen, difunktionellen Estern von Phthalsäure, difunktionellen Estern von Methacrylsäure, polyfunktionellen Estern von Acrylsäure, Dienen, Trienen, Tetraenen und N-Methylen-bis-acrylamid bestehenden Gruppe.

24. Zusammensetzung nach Anspruch 23, worin die Emulsion 0,1 bis 1 Gew.-% des vernetzenden Monomers, bezogen auf das Gesamtgewicht an Monomer, umfasst.

25. Zusammensetzung nach Anspruch 20, worin das assoziative Monomer ausgewählt ist aus der Gruppe, bestehend aus Urethan-Umsetzungsprodukten eines monoethylenisch ungesättigten Isocyanats und nichtionischen oberflächenaktiven Mitteln, umfassend C₁-C₄-Alkoxy-terminierte, Block-Copolymeren von 1,2-Butylenoxid und 1,2-Ethylenoxid, einem ethylenisch ungesättigten copolymerisierbaren oberflächenaktiven Monomer, erhalten durch Kondensieren eines nichtionischen oberflächenaktiven Mittels mit Methylensuccinsäure, einem oberflächenaktiven Monomer, ausgewählt aus dem Harnstoff-Umsetzungsprodukt eines monoethylenisch ungesättigten Monoisocyanats mit einem nichtionischen oberflächenaktiven Mittel mit einer Amin-Funktionalität und einem nichtionischen Urethanmonomer, welches das Urethan-Umsetzungsprodukt eines einwertigen nichtionischen oberflächenaktiven Mittels mit einem monoethylenisch ungesättigten Monoisocyanat darstellt.

26. Zusammensetzung nach Anspruch 20, worin die Emulsion 0,1 bis 10 Gew.-% des assoziativen Monomers, bezogen auf das Gesamtgewicht an Monomer, umfasst.

27. Zusammensetzung nach Anspruch 20, worin das Monomer (b) ausgewählt ist aus der aus N,N-Dimethylaminoethylmethacrylat, N,N-Diethylaminoethylacrylat, N,N-Diethylaminoethylmethacrylat, N-t-Butylaminoethylacrylat, N-t-Butylaminoethylmethacrylat, N, N-Dimethylaminopropylacrylamid, N,N-Dimethylaminopropylmethacrylamid, N,N-Diethylaminopropylacrylamid und N,N-Diethylaminopropylmethacrylamid bestehenden Gruppe.

28. Zusammensetzung nach Anspruch 20 im wesentlichen bestehend aus der stabilen Emulsion.

## Revendications

1. Composition aqueuse ayant un pH inférieur ou égal à 11, comprenant :
une quantité épaississante d'une émulsion stable d'un polymère, ladite émulsion étant préparée par une polymérisation en émulsion en une étape de monomères choisis dans le groupe consistant en
5 à 80 pour cent en poids d'un monomère acrylate (a) choisi dans le groupe consistant en un ester d'alkyle en C₂ à C₆ d'acide acrylique et un ester d'alkyle en C₁ à C₆ d'acide méthacrylique,
5 à 80 pour cent en poids d'un monomère (b) choisi dans le groupe consistant en un composé hétérocyclique à substituant vinyle contenant au moins un atome de soufre ou d'azote, un monomère (méth)acrylamide, un (méth)acrylate de mono- ou di-(alkyle en C₁ à C₄)amino-(alkyle en C₁ à C₄) et un (méth)acrylamide de mono- ou di(alkyle en C₁ à C₄)amino-(alkyle en C₁ à C₄),
0 à 2 pour cent en poids d'un monomère de réticulation (c), et
0 à 30 pour cent en poids d'un monomère associatif (d),
tous les pourcentages étant basés sur le poids total des monomères,
dans laquelle ladite polymérisation en émulsion est effectuée en présence d'eau, d'un premier surfactant en des quantités efficaces pour émulsionner le polymère dans l'eau, d'un initiateur radicalaire et de 0,5 à 20 pour cent en poids d'un alcool choisi dans le groupe consistant en un alcool monohydroxylique en C₂ à C₁₂, linéaire ou ramifié et un alcool polyhydroxylique non polymère, sur la base du poids total de ladite émulsion, ladite polymérisation en émulsion en une étape étant effectuée pratiquement en l'absence d'un stabilisant colloïdal polymère ; et
une quantité efficace minimale d'un ingrédient actif choisi dans le groupe consistant en un acide et un second surfactant.

2. Composition suivant la revendication 1, dans laquelle l'émulsion comprend 15 à 40 pour cent en poids du polymère, sur la base du poids total de ladite émulsion.

3. Composition suivant la revendication 1, dans laquelle l'émulsion comprend 0,1 à 5 pour cent en poids du premier surfactant, sur la base du poids total des monomères, ledit premier surfactant étant choisi dans le groupe consistant en des surfactants anioniques, cationiques, non ioniques, amphotères et zwittérioniques.

4. Composition suivant la revendication 1, dans laquelle le monomère de réticulation est choisi dans le groupe consistant en monomères aromatiques à substituants vinyle multiples, monomères alicycliques choisis dans le groupe consistant en cycloparaffines et cyclooléfines, esters difonctionnels d'acide phtalique, esters difonctionnels d'acide méthacrylique, esters multifonctionnels d'acide acrylique, diènes, triènes, tétraènes et N-méthylène-bis-acrylamide.

5. Composition suivant la revendication 4, dans laquelle l'émulsion comprend 0,1 à 1 pour cent en poids du monomère de réticulation, sur la base du poids total des monomères.

6. Composition suivant la revendication 1, dans laquelle le monomère associatif est choisi dans le groupe consistant en produits de réaction du type uréthanne d'un isocyanate à insaturation monoéthylénique et de surfactants non-oniques comprenant des copolymères séquencés, à terminaison alkoxy en C₁ à C₄, d'oxyde de 1,2-butylène et d'oxyde de 1,2-éthylène, un monomère surfactant copolymérisable à insaturation éthylénique obtenu par condensation d'un surfactant non ionique avec l'acide méthylène succinique, un monomère surfactant choisi entre le produit de réaction du type urée d'un monoisocyanate à insaturation monoéthylénique avec un surfactant non ionique ayant une fonctionnalité amine, et un monomère uréthanne non ionique qui est le produit de réaction du type uréthanne d'un surfactant non ionique monohydroxylique avec un isocyanate à insaturation monoéthylénique.

7. Composition suivant la revendication 6, dans laquelle l'émulsion comprend 0,1 à 10 pour cent en poids du monomère associatif, sur la base du poids total des monomères.

8. Composition suivant la revendication 1, dans laquelle le monomère (b) est choisi dans le groupe consistant en le méthacrylate de N,N-diméthylaminoéthyle, l'acrylate de N,N-diéthylaminoéthyle, le méthacrylate de N,N-diéthylaminoéthyle, l'acrylate de N-tertio-butylaminoéthyle, le méthacrylate de N-tertio-butylaminoéthyle, le N,N-diméthylaminopropylacrylamide, le N,N-diméthylaminopropylméthacrylamide, le N,N-diéthylaminopropylacrylamide et le N,N-diéthylaminopropylméthacrylamide.

9. Composition suivant la revendication 1, dans laquelle le second surfactant est choisi dans le groupe consistant en des surfactants anioniques et des surfactants non ioniques.

10. Composition suivant la revendication 9, dans laquelle le second surfactant est présent en une quantité nettoyante minimale, allant jusqu'à 95 pour cent en poids de matière active, sur la base du poids total de ladite composition.

11. Composition suivant la revendication 10, comprenant 2 à 95 pour cent en poids de matière active du second surfactant, sur la base du poids total de la composition épaissie.

12. Composition suivant la revendication 1, comprenant 0,1 à 95 pour cent en poids de matière active du second surfactant, sur la base du poids total de la composition épaissie.

13. Composition suivant la revendication 1, dans laquelle l'acide est présent en une quantité minimale nettoyante, allant jusqu'à 50 pour cent en poids de matière active, sur la base du poids total de ladite composition.

14. Composition suivant la revendication 13, comprenant 2 à 50 pour cent en poids de matière active de l'acide, sur la base du poids total de ladite composition.

15. Composition suivant la revendication 13, comprenant le second surfactant en des quantités efficaces pour améliorer l'efficacité d'épaississement de l'émulsion.

16. Composition suivant la revendication 1, comprenant 0,1 à 50 pour cent en poids de matière active de l'acide, sur la base du poids total de ladite composition.

17. Composition suivant la revendication 1, comprenant 0,5 à environ 20 pour cent en poids sec du polymère, sur la base du poids total de ladite composition.

18. Composition suivant la revendication 1, dans laquelle l'acide est choisi dans le groupe consistant en les acides citrique, sulfurique, hydrochloridique, phosphorique, acétique, hydroxyacétique et sulfamique.

19. Composition suivant la revendication 1, dans laquelle le second surfactant est choisi dans le groupe consistant en le laurylsulfate de sodium, des (di)alkylsulfosuccinates, des alkylsulfonates, des alkylphosphates, des alkyléthoxylates et des alkylaryléthoxylates.

20. Composition destinée à être utilisée comme modificateur de rhéologie dans des milieux aqueux ayant un pH inférieur ou égal à 11, ladite composition comprenant :
une émulsion stable d'un polymère, ladite émulsion étant préparée par une polymérisation en émulsion en une étape de monomères choisis dans le groupe consistant en
5 à 80 pour cent en poids d'un monomère acrylate (a) choisi dans le groupe consistant en un ester d'alkyle en C₂ à C₆ d'acide acrylique et un ester d'alkyle en C₁ à C₆ d'acide méthacrylique,
5 à 80 pour cent en poids d'un monomère (b) choisi dans le groupe consistant en un composé hétérocyclique à substituant vinyle contenant au moins un atome d'azote ou de soufre, un monomère (méth)acrylamide, un (méth)acrylate de mono- ou di-(alkyle en C₁ à C₄)amino-(alkyle en C₁ à C₄) et un (méth)acrylamide de mono- ou di(alkyle en C₁ à C₄)amino-(alkyle en C₁ à C₄),
0 à 2 pour cent en poids d'un monomère de réticulation (c) ; et
0 à 30 pour cent en poids d'un monomère associatif (d),
tous les pourcentages étant basés sur le poids total des monomères,
dans laquelle ladite polymérisation en émulsion est effectuée en présence d'eau, d'un premier surfactant en des quantités efficaces pour émulsionner le polymère dans l'eau, d'un initiateur radicalaire et de 0,5 à 20 pour cent en poids d'un alcool choisi dans le groupe consistant en un alcool monohydroxylique en C₂ à C₁₂, linéaire ou ramifié et un alcool polyhydroxylique non polymère, sur la base du poids total de ladite émulsion, ladite polymérisation en émulsion en une étape étant effectuée pratiquement en l'absence d'un stabilisant colloïdal polymère.

21. Composition suivant la revendication 20, dans laquelle l'émulsion comprend 15 à 40 pour cent en poids du polymère, sur la base du poids total de ladite émulsion.

22. Composition suivant la revendication 20, dans laquelle l'émulsion comprend 0,1 à 5 pour cent en poids du surfactant, sur la base du poids total des monomères, ledit surfactant étant choisi dans le groupe consistant en des surfactants anioniques, cationiques, non ioniques, amphotères et zwittérioniques.

23. Composition suivant la revendication 20, dans laquelle le monomère de réticulation est choisi dans le groupe consistant en monomères aromatiques à substituants vinyle multiples, monomères alicycliques choisis dans le groupe consistant en cycloparaffines et cyclooléfines, esters difonctionnels d'acide phtalique, esters difonctionnels d'acide méthacrylique, esters multifonctionnels d'acide acrylique, diènes, triènes, tétraènes et N-méthylène-bis-acrylamide.

24. Composition suivant la revendication 23; dans laquelle l'émulsion comprend 0,1 à 1 pour cent en poids du monomère de réticulation, sur la base du poids total des monomères.

25. Composition suivant la revendication 20, dans laquelle le monomère associatif est choisi dans le groupe consistant en des produits de réaction du type uréthanne d'un isocyanate à insaturation monoéthylénique et de surfactants non-ioniques comprenant des copolymères séquencés, à terminaison alkoxy en C₁ à C₄, d'oxyde de 1,2-butylène et d'oxyde de 1,2-éthylène, un monomère surfactant copolymérisable à insaturation éthylénique obtenu par condensation d'un surfactant non ionique avec l'acide méthylène succinique, un monomère surfactant choisi entre le produit de réaction du type urée d'un monoisocyanate à insaturation monoéthylénique avec un surfactant non ionique ayant une fonctionnalité amine, et un monomère uréthanne non ionique qui est le produit de réaction du type uréthanne d'un surfactant non ionique monohydroxylique avec un monoisocyanate à insaturation monoéthylénique.

26. Composition suivant la revendication 20, dans laquelle l'émulsion comprend 0,1 à 10 pour cent en poids du monomère associatif, sur la base du poids total des monomères.

27. Composition suivant la revendication 20, dans laquelle le monomère (b) est choisi dans le groupe consistant en le méthacrylate de N,N-diméthylaminoéthyle, l'acrylate de N,N-diéthylaminoéthyle, le méthacrylate de N,N-diéthylaminoéthyle, l'acrylate de N-tertio-butylaminoéthyle, le méthacrylate de N-tertio-butylaminoéthyle, le N,N-diméthylaminopropylacrylamide, le N,N-diméthylaminopropylméthacrylamide, le N,N-diéthylaminopropylacrylamide et le N,N-diéthylaminopropylméthacrylamide.

28. Composition suivant la revendication 20, consistant essentiellement en l'émulsion stable.
